Europäisches Patentamt

European Patent Office

Office européen des brevets

(19)

(11) Publication number: **0 226 588 B1**

(12)

# EUROPEAN PATENT SPECIFICATION
## published in accordance with Art. 158(3) EPC

(45) Date of publication of patent specification: **20.03.91**

(51) Int. Cl.⁵: **A61K 31/44, A61K 9/52**

(21) Application number: **85904808.4**

(22) Date of filing: **04.09.85**

(86) International application number:
**PCT/EP85/00481**

(87) International publication number:
**WO 86/01717 (27.03.86 86/07)**

(54) **Process for preparation of a solid form for oral use based on nifedipine with controlled release of the active substance.**

(30) Priority: **14.09.84 IT 2267884**

(43) Date of publication of application:
**01.07.87 Bulletin 87/27**

(45) Publication of the grant of the patent:
**20.03.91 Bulletin 91/12**

(84) Designated Contracting States:
**AT BE CH DE FR GB IT LI LU NL SE**

(56) References cited:
**EP-A- 0 047 899**
**FR-A- 2 453 642**

**Chemical Abstracts, vol. 97, nr. 10, 6 September 1982, Columbus, Ohio, USA Page 423, column 2, abstract nr. 78923a**

(73) Proprietor: **PHARMATEC S.p.A.**
**Via Carducci, 35**
**I-20090 Trezzano Sul Naviglio Milano(IT)**

(72) Inventor: **GIUDICE, Vittorio**
**2, Via Orlando da Tresseno**
**I-20127 Milano(IT)**
Inventor: **RAVELLI, Vittorino**
**189, Via Palmanova**
**I-20132 Milano(IT)**

(74) Representative: **de Pasquale, Carlo et al**
**Via Carlo Ravizza 53**
**I-20149 Milano(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Chemical Abstracts, vol. 98, nr. 16, 18 April 1983, Columbus, Ohio, USA J. Sugimoto et al.:"Stability and bio-availability of nifedipine in fine granules", page 363, column 2, abstract nr. 132212d

Chemical Abstracts, vol. 99, nr. 6, 8 August 1983, Columbus, Ohio, USA, page 323, column 1, abstract nr. 43571y

**Description**

The present invention relates to a process for preparation of a solid form for oral use based on nifedipine with controlled release of the active substance.

Nifedipine belongs to the group of substances having a calcium antagonistic activity. Its chemical name according to the IUPAC nomenclature is 4-(2'-nitropyridine)-3,5-dicarboxylic acid methyl ester.

Nifedipine is known to be a substance with high pharmacological activity and its mechanism of action is known and described in the literature. From literature it is also known that nifedipine, administered in such a way as to give rise to plasma levels in the order of 100-200 g/ml in a short time, is effective to prevent and treat attacks of angina pectoris, while at blood concentrations of 20-80 g/ml carries out an effective activity in the treatment of arterial hypertension.

Already when taken alone, nifedipine has a low solubility in aqueous solvents and in the range of physiological pH. Use of nifedipine in traditional pharmaceutical forms tends to further increase this feature and in many cases it gives rise to preparations with a very long time of dissolution of the active substance and great variability from one preparation to another.

This causes a variability of absorption of the active substance by the organism, both as values of blood concentration versus time, and total bioavailability, i.e. total amount of nifedipine absorbed in a given time interval.

Improvements in this respect were obtained by using nifedipine substance having a sufficiently high specific surface area of crystals (see German published patent application DE 3033919 A1), more particularly between 0.5 and 6 $m^2/g$, or by increasing solubility of nifedipine by forming co-precipitates with auxiliary substances (see European published patent application EP 0001247).

These and other systems are clearly effective to obtain pharmaceutical forms based on nifedipine, adapted to give a prompt release and consequent absorption of the active substance and therefore suitable for treating angina pectoris with nifedipine.

The problem of making a pharmaceutical solid form for oral use with a delayed and sustained release of a satisfactory amount of nifedipine remains however unsolved, as the retard administration forms presently available have poor release characteristics in view of the above said poor solubility of the active substance and therefore they show a rather unsatisfactory bioavailability and a considerable lack of time uniformity.

The object of the present invention is therefore the preparation of a pharmaceutical solid form for oral use, adapted to release nifedipine in a time controlled and gradual way, in order to obtain a continuous and prolonged absorption of the active substance by the human being, so as to warrant plasma levels effective for treating arterial hypertension.

For this purpose, nifedipine is used in the form of crystal having a specific surface area between 0.1 and 0.4 $m^2$ /g. The substance is mixed in a suitable equipment with an equal amount by weight of an auxiliary vehicle substance. Among the auxiliary vehicle substances adapted to make pharmaceutical solid forms lactose, corn starch, modified starch are preferred.

Such a mixture is applied on spheroids made of inert substances such as sucrose and starch by using suitable binders, like low molecular weight polyvinylpyrrolidone solutions or polyoxyethyleneglycol solutions, in coating pans.

One of the main problems arising from this type of treatment is connected with the relatively poor distribution of the powder spread on the mass of inert microgranules, which causes an uneven dosage of nifedipine as a direct consequence.

Such an uneven distribution is also reflected, in view of the very low nifedipine solubility, in obtaining batches with very different dissolution speeds and with uneven dissolution speed inside the same batch.

Surprisingly good results were achieved by using particular powder homogenization and deposition techniques so as to obtain a uniform and homogeneous distribution of the active substance powder on the microgranules.

The results of uniformity tests for nifedipine contents on pellets confirmed the above, giving standard deviation values which can be considered similar to the pattern of granulometric distribution of inert microgranules, and therefore to their surface area.

The obtained values of in vitro release of the active substance are moreover reproducible from batch to batch and are homogeneous inside the same batch.

The microgranules so obtained were dosed in hard gelatine capsules in an amount corresponding to 20 mg of nifedipine.

Subsequent tests of bioavailability in human beings showed that the preparations of the present invention allow to obtain plasma levels of nifedipine reaching 20 µg/ml just after 30 minutes and are maintained between 20 and 80µg/ml for 10 hours or more. The bioavailability of these preparations is

3

higher than 90% when compared with a prompt absorption form.

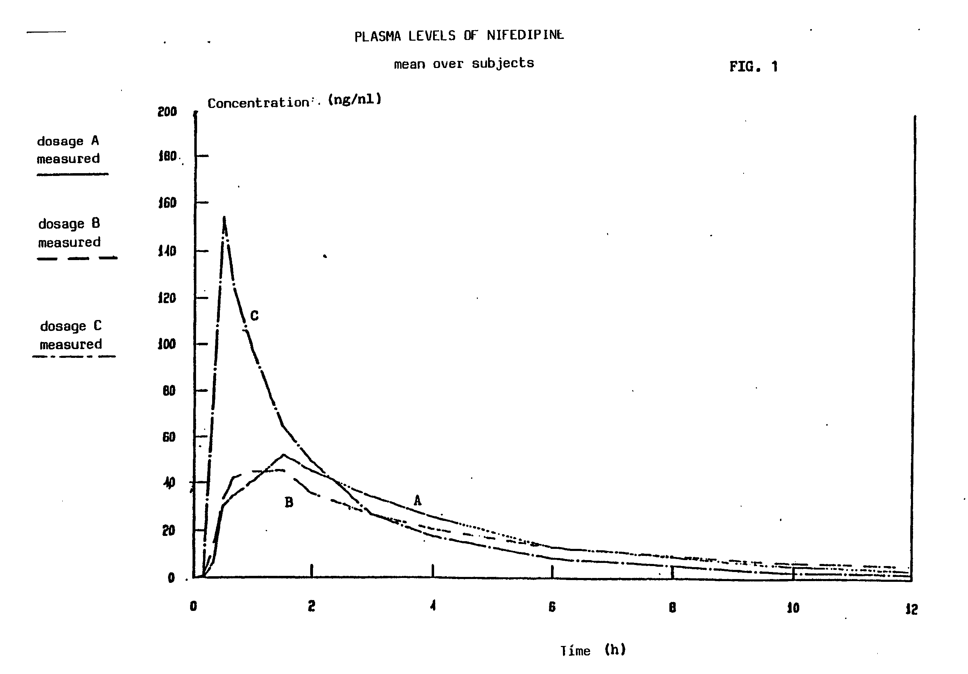

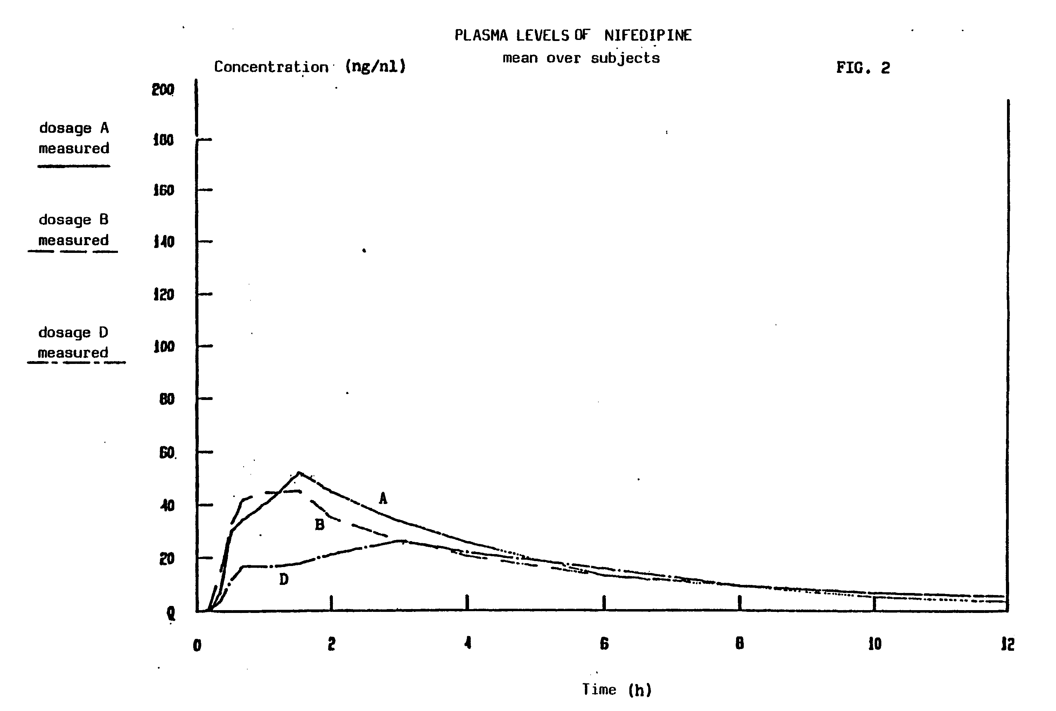

The accompanying drawings show the patterns of the plasma levels of the preparations made according to the present invention, compared with that of a normal administration form (Fig. 1) and that of a retard form presently available on the market (Fig. 2).

As a further confirmation of the favorable characteristics of the preparations made according to the present invention, the following Examples A and B illustrate the production of such a formulation.

## EXAMPLE A

2 kg of nifedipine raw material, having a specific surface area between 0.1 and 0.4m²/g, are mixed with 2kg of lactose having a granulometry between 10 and 80μm, using a rotary cylinder mixer. In a coating machine having a stainless steel pan of 600 mm diameter, 8 kg of microgranules consisting of sucrose and corn starch with a granulometry between 0.790 and 0.910 mm are loaded.

The nifedipine powder mixture is loaded in a suitable container provided with feeding suction device, pneumatic vibrator and regulation pressure gauge.

Rotation of the coating pan is started at a speed of 16 rpm and microgranules are wetted with 50 g of a 10% solution of polyvinylpyrrolidone (k 30) in ethyl alcohol. An airless type pump is used to this purpose.

On the rotating wet mass 50 g of nifedipine powder are then sprayed by means of suitable pulverization devices, fed by the above said suitable container. Operations are alternately repeated 80 times in total.

At this point while the coating pan is always rotating, the microgranules are wetted with 200 g of a 12.5% solution of polymethacrylates (Eudragit L®) in ethanol: acetone.

On the rotating wet mass 100g of talc are then sprayed by using the same procedure indicated for the nifedipine-lactose mixture. The microgranules so obtained show a good uniformity of nifedipine contents.

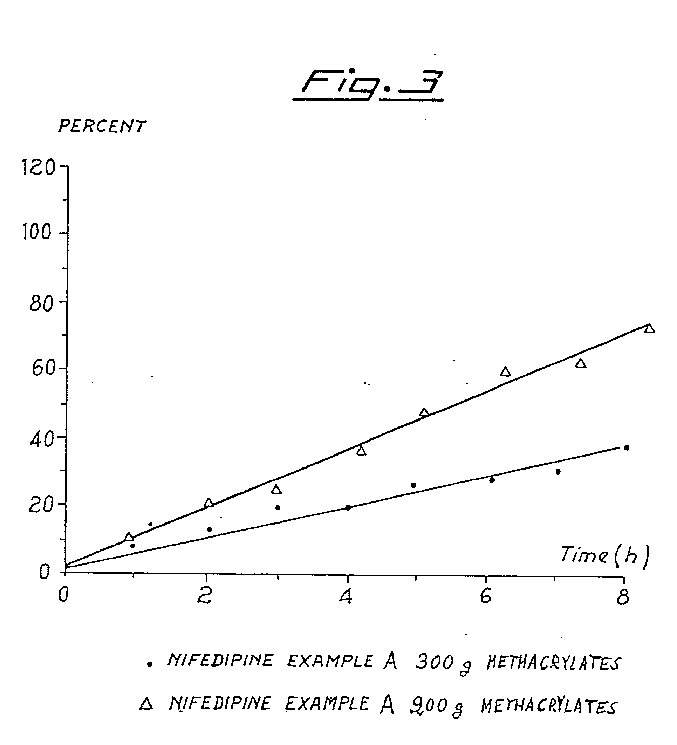

Test of in vitro release velocity with the continuous flow method shows a zero order release pattern distinctly modified in comparison with the pure substance and velocity of nifedipine release from microgranules decreases consistently with the increase of the amount of polymethacrylates (Fig. 3).

This relationship is confirmed by the results obtained through a measurement of blood levels of nifedipine in human beings, after administration of several formulations corresponding to the above preparation.

## EXAMPLE B

In a coating machine provided with a stainless steel pan of 600 mm diameter, 10 kg of microgranules consisting of sucrose and corn starch with a granulometry between 1.0 and 1.4mm are loaded.

In two separate suitable containers, 4 kg of nifedipine having a specific surface are between 0.1 and 0.4 m²/g and 6 kg of corn starch with a granulometry between 20 and 100μm are respectively loaded.

Rotation of the coating pan is started at a speed of 16 rpm and the microgranules are wetted with 100g of a 10% solution of polyoxyethyleneglycol 4000 in ethyl alcohol. An airless type pump is used for this purpose.

Then on the rotating wet mass 200 g of nifedipine and 300 g of corn starch are sprayed with two pulverization devices, each connected to one of said containers. These operations are carried out at the same time and the procedure is repeated 20 times.

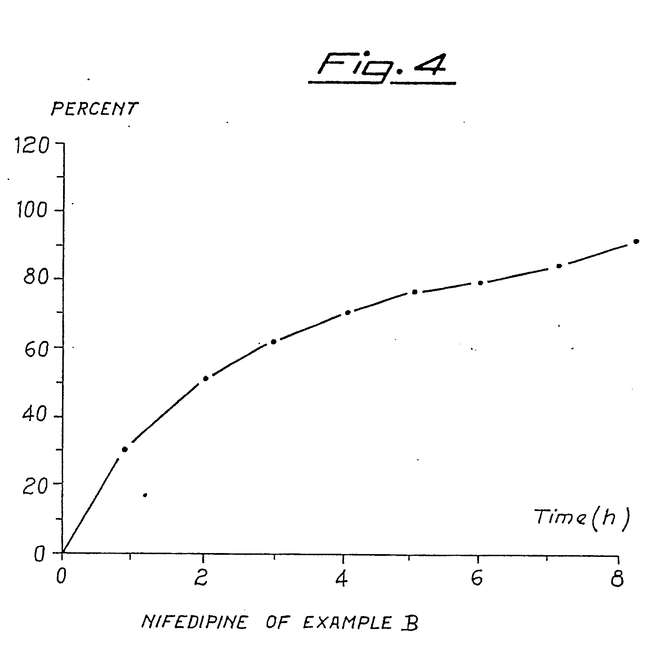

Test of in vitro release velocity shows a first order release pattern with a total amount of released nifedipine higher than 80% in 8 hours (Fig. 4). Control of uniformity of nifedipine contents confirms the uniform distribution of the active substance on the microgranules.

Indeed, checking the patterns of plasma levels A and B corresponding to the preparations of the above Examples, it is to be noted how they are well differentiated not only in respect of curve C (Fig. 1) of normal nifedipine showing a sharp rise in the first minutes and then likewise rapid decrease, suitable for treating an attack of angina pectoris, but also in respect of curve D (Fig. 2) where it is to be noted that the bioavailability of a retard form prepared with conventional methods is constantly poor, while bioavailability of forms A and B according to the present invention is for superior, especially in the first four hours, and being at the center of the range of therapeutically active plasma levels, it achieves optimal blood levels effective for treating arterial hypertension.

It is therefore clear that the administration forms prepared with the process according to the present invention allow an effective treatment of arterial hypertension, in view of the controlled release of the active substance with reproducible blood levels constantly falling in the range of bioavailability suitable for treating said disorder.

In the light of the results obtained, showing for preparations A and B a clear maintenance for a long time of therapeutically advantageous blood levels, it was considered useful to carry out a further pharmacokinetic study at repeated dosages, thus simulating the posolcgic scheme suitable for an antihypertensive treatment for a period of five days.

To this purpose preparation A was chosen and the results of such an experimentation, always carried out in comparison with a conventional retard form already available on the market and with nifedipine in quick release form, fully confirm the results of the preceding tests, again showing for preparation A a better bioavailability in respect of the above mentioned conventional product, without causing however any phenomenon of accumulation with time.

The study was conduced on twelve subjects, of which four female and eight male subjects, whose characteristics are set forth in the following table:

|              | mean  | standard deviation |
|--------------|-------|--------------------|
| age (years)  | 26.5  | 5.6                |
| height (cm)  | 171.8 | 8.8                |
| weight (kg)  | 67.5  | 10.3               |

The following tables 1-3 show the blood levels measured on patients at day 1 of treatment, then during the accumulation phase (day 2,3 and 4) and finally at day 5 in the elimination phase. In the tables, treatment A of days 1-4 and D of day 5 is that effected with one capsule of 20 mg of nifedipine of the present invention, treatment B of days 1-4 and E of day 5 is that effected with two capsules of 10 mg of quick release nifedipine (Adalat), and treatment C of days 1-4 and F of day 5 is that effected with a tablet of 20 mg of delayed nifedipine available on the market (Adalat-Retard). Plasma level concentrations are expressed in ng/ml, while the indicative symbols of the lines of data have the following meanings: mean = mean value; sdev = standard deviation; sem = standard error of the mean. Times are expressed in minutes, hours and days as indicated.

EP 0 226 588 B1

## TABLE 1

### PLASMA LEVELS MEASURED AT DAY 1

| Time | 0h | 10 min. | 20 min. | 30 min. | 40 min. | 1 h | 1h30 min. | 2h |
|---|---|---|---|---|---|---|---|---|
| **Treatment A** | | | | | | | | |
| mean | 0 | 0 | 17.632 | 34.743 | 41.684 | 46.481 | 46.996 | 50.936 |
| sdev | 0 | 0 | 22.039 | 35.051 | 36.645 | 34.354 | 28.044 | 27.532 |
| sem | 0 | 0 | 6.362 | 10.118 | 10.579 | 9.917 | 8.096 | 7.948 |
| **Treatment B** | | | | | | | | |
| mean | 0 | 1.391 | 124.013 | 164.99 | 150.719 | 110.47 | 79.74 | 56.942 |
| sdev | 0 | 4.818 | 103.359 | 47.881 | 31.938 | 21.044 | 22.999 | 15.439 |
| sem | 0 | 1.391 | 29.837 | 13.822 | 9.22 | 6.075 | 6.639 | 4.457 |
| **Treatment C** | | | | | | | | |
| mean | 0 | 0 | 11.339 | 25.163 | 27.733 | 31.453 | 32.133 | 28.124 |
| sdev | 0 | 0 | 8.677 | 13.505 | 13.931 | 15.677 | 18.085 | 14.792 |
| sem | 0 | 0 | 2.505 | 3.898 | 4.022 | 4.526 | 5.221 | 4.27 |

TABLE 1 CONTINUED

PLASMA LEVELS MEASURED AT DAY 1

| tempo | 3h | 4h | 6h | 8h | 10h | 12h | 24h |
|---|---|---|---|---|---|---|---|
| Treatment A | | | | | | | |
| mean | 52.758 | 37.31 | 22.014 | 14.137 | 8.548 | 5.493 | 0.636 |
| sdev | 18.616 | 13.794 | 10.658 | 6.272 | 5.616 | 3.796 | 1.591 |
| sem | 5.374 | 3.982 | 3.214 | 1.81 | 1.621 | 1.096 | 0.459 |
| Treatment B | | | | | | | |
| mean | 40.68 | 26.884 | 14.635 | 8.947 | 5.772 | 2.931 | 0 |
| sdev | 14.939 | 9.017 | 5.782 | 3.39 | 2.791 | 2.468 | 0 |
| sem | 4.312 | 2.603 | 1.669 | 1.022 | 0.806 | 0.713 | 0 |
| Treatment C | | | | | | | |
| mean | 27.749 | 23.623 | 16.93 | 13.998 | 10.207 | 8.247 | 1.557 |
| sdev | 10.614 | 8 | 7.379 | 6.542 | 4.514 | 4.198 | 1.673 |
| sem | 3.064 | 2.309 | 2.13 | 1.888 | 1.361 | 1.212 | 0.483 |

EP 0 226 588 B1

TABLE 2

## PLASMA LEVELS DURING THE ACCUMULATION PHASE

| Time (days). | 2 | 3 | 4 |
|---|---|---|---|
| **Treatment A** | | | |
| mean | 10.427 | 10.125 | 10.175 |
| sdev | 5.399 | 6.847 | 4.473 |
| sem | 1.559 | 1.976 | 1.291 |
| **Treatment B** | | | |
| mean | 5.308 | 3.745 | 6.693 |
| sdev | 3.902 | 1.979 | 6.346 |
| sem | 1.126 | 0.571 | 1.832 |
| **Treatment C** | | | |
| mean | 10.073 | 10.97 | 11.383 |
| sdev | 2.926 | 5.759 | 5.445 |
| sem | 0.845 | 1.736 | 1.572 |

## TABLE 3

## PLASMA LEVELS MEASURED AT DAY 5

| Time | 0h | 10 min. | 20 min. | 30 min. | 40 min. | 1h | 1h30 min. | 2h |
|---|---|---|---|---|---|---|---|---|
| **Treatment D** | | | | | | | | |
| mean | 10.177 | 9.581 | 18.157 | 37.318 | 39.087 | 53.109 | 62.313 | 61.113 |
| sdev | 4.477 | 4.439 | 11.595 | 25.319 | 26.853 | 27.402 | 27.078 | 19.159 |
| sem | 1.292 | 1.338 | 3.496 | 7.309 | 8.097 | 7.91 | 7.817 | 5.531 |
| **Treatment E** | | | | | | | | |
| mean | 6.694 | 12.15 | 190.655 | 182.275 | 151.818 | 108.3 | 81.522 | 61.133 |
| sdev | 6.344 | 10.394 | 91.761 | 77.762 | 62.372 | 34.238 | 27.334 | 15.75 |
| sem | 1.831 | 3.001 | 26.489 | 22.448 | 18.806 | 9.884 | 7.891 | 4.547 |
| **Treatment F** | | | | | | | | |
| mean | 11.387 | 10.644 | 17.538 | 30.727 | 34.421 | 38.155 | 36.5 | 36.593 |
| sdev | 5.449 | 4.873 | 8.53 | 16.059 | 14.969 | 12.153 | 12.433 | 13.387 |
| sem | 1.573 | 1.407 | 2.462 | 4.636 | 4.321 | 3.508 | 3.589 | 3.864 |

EP 0 226 588 B1

# TABLE 3 CONTINUED

## PLASMA LEVELS MEASURED AT DAY 5

| Time | 3h | 4h | 6h | 8h | 10h | 12h | 24h | 48h |
|------|-----|-----|-----|-----|-----|-----|-----|-----|
| **Treatment D** | | | | | | | | |
| mean | 51,102 | 35,56 | 20.341 | 13.446 | 9.091 | 6,131 | 0,222 | 0 |
| sdev | 14,661 | 9,665 | 5.389 | 4.361 | 4.647 | 3.512 | 0.768 | 0 |
| sem | 4,232 | 2,79 | 1.556 | 1.259 | 1.401 | 1,059 | 0.222 | 0 |
| **Treatment E** | | | | | | | | |
| mean | 37.703 | 26.418 | 15.106 | 9,054 | 5.481 | 3.537 | 0 | 0 |
| sdev | 10.34 | 6.18 | 4.918 | 4,103 | 3,27 | 2,347 | 0 | 0 |
| sem | 2.985 | 1.784 | 1.42 | 1.184 | 0.944 | 0,677 | 0 | 0 |
| **Treatment F** | | | | | | | | |
| mean | 31.025 | 26,748 | 18.899 | 15,39 | 11,816 | 9,781 | 2.58 | 0 |
| sdev | 10.911 | 9.328 | 8.556 | 7,177 | 5.059 | 3.502 | 1,682 | 0 |
| sem | 3,15 | 2,693 | 2.47 | 2,072 | 1,46 | 1,011 | 0,486 | 0 |

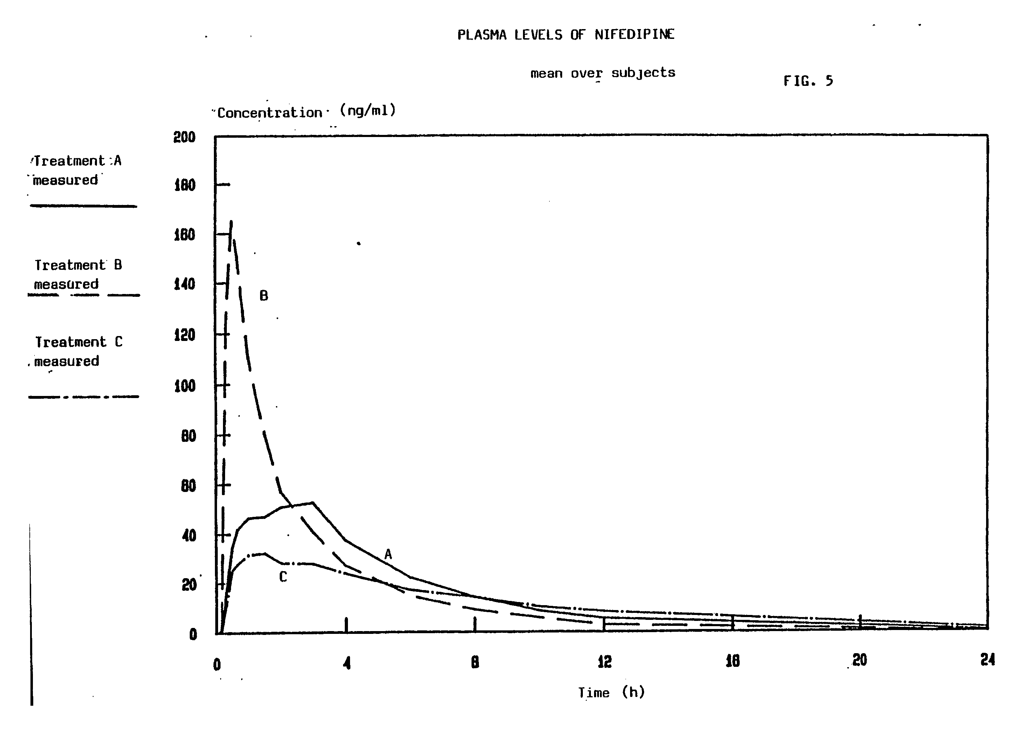

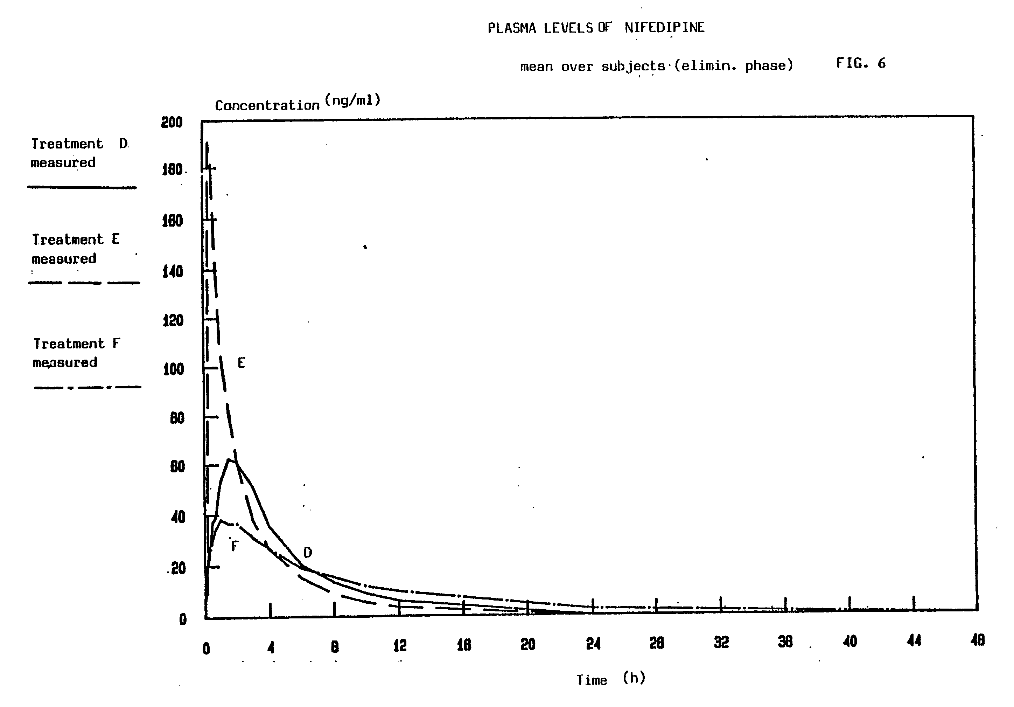

On the basis of the data measured and shown in the preceding tables 1-3, mean curves of plasma levels of nifedipine at day 1 (Fig. 5) and at day 5 (Fig. 6) were plotted and it is possible to note even visually that there are no phenomena of accumulation with time of the pharmaceutical preparation according to the present invention.

The following tables 4 and 5 show the pharmacokinetic parameters calculated from the plasma levels of nifedipine measured after treatment, wherein treatments A, B, C, D, E, F have the same meanings as previously indicated, and the symbols of the various parameters are corresponding to the following definitions:

| | |
|---|---|
| AUC - 1: | trapezium rule, O-last sampling (SD), dosage interval (MD) |
| AUG - 3: | model curve, O-infinity (SD), dosage interval (MD) |
| C max: | peak concentration |
| C max(m): | measured peak concentration |
| T max: | peak time |
| T max(m): | observed peak time |
| lag-time : | start time of absorption |
| ke : | elimination rate constant |
| t1/2 : | elimination half-life |
| mrt: | mean residence time |
| t1/2 alpha: | alpha half-life |
| t1/2 beta: | beta half-life |

In the table 4 the pharmacokinetic parameters calculated at day 1 are shown, while in table 5 the same parameters at day 5 are shown.

It may be noted also from these further tables that the pharmaceutical preparation according to the present invention has superior characteristics in comparison with the nifedipine formulations presently available on the market.

## TABLE 4

### PHARMACOKINETIC PARAMETERS AT DAY 1

| Parameter | AUC - 1 | AUC - 3 | $C_{max}$ | $C_{max}$ (m) | $T_{max}$ | $T_{max}$ (m) |
|---|---|---|---|---|---|---|
| **Treatment A** | | | | | | |
| mean | 317.814 | 337.75 | 81.1821 | 75.2575 | 1.95621 | 1.91667 |
| sdev | 107.601 | 117.849 | 29.5296 | 22.1234 | 1.07462 | 1.04083 |
| sem | 31.0616 | 34.0201 | 8.52447 | 6.38649 | 0.310215 | 0.300463 |
| unit | hxng/ml | hxng/ml | ng/ml | ng/ml | h | h |
| **Treatment B** | | | | | | |
| mean | 356.735 | 376.218 | 201.258 | 177.598 | 0.475803 | 0.5 |
| sdev | 85.656 | 90.6711 | 60.6552 | 53.4101 | 0.124258 | 0.142134 |
| sem | 24.7268 | 26.1745 | 17.5096 | 15.4182 | 0.03587 | 0.041031 |
| unit | hxng/ml | hxng/ml | ng/ml | ng/ml | h | h |
| **Treatment C** | | | | | | |
| mean | 256.17 | 304.479 | 40.3367 | 40.2008 | 1.6083 | 1.54167 |
| sdev | 96.3563 | 98.8216 | 17.4381 | 16.2849 | 1.26191 | 1.13735 |
| sem | 27.8157 | 28.5273 | 5.03394 | 4.70104 | 0.364281 | 0.328324 |
| unit | hxng/ml | hxng/ml | ng/ml | ng/ml | h | h |

EP 0 226 588 B1

TABLE 4 CONTINUED

## PHARMACOKINETIC PARAMETERS AT DAY 1

| Parameter | lag-time | ke | t1/2 | mrt | t1/2 alfa | t1/2 beta |
|---|---|---|---|---|---|---|
| **Treatment A** | | | | | | |
| mean | 0.525066 | 1.04252 | 1,6814 | 5,30887 | 0.301548 | 3.51796 |
| sdev | 0,569852 | 1.42199 | 1.16067 | 2.13099 | 0.325898 | 1,92126 |
| sem | 0.164502 | 0.410493 | 0.335058 | 0.615163 | 0.123178 | 0.726168 |
| unit | h | 1/h | h | h | h | h |
| **Treatment B** | | | | | | |
| mean | 0.137192 | 0.968181 | 0.960704 | 3,13586 | 0.339462 | 2.55556 |
| sdev | 0.141495 | 0.767975 | 0,413819 | 0.89595 | 0.254272 | 0,946419 |
| sem | 0.040846 | 0.221695 | 0,119459 | 0,258639 | 0.073402 | 0.273208 |
| unit | h | 1/h | h | h | h | h |
| **Treatment C** | | | | | | |
| mean | 0.222035 | 0.574286 | 3.25592 | 10.3735 | 0.656811 | 9,37473 |
| sdev | 0,124974 | 0,68202 | 2.18483 | 4.56167 | 0.96815 | 4,60459 |
| sem | 0.036077 | 0.196882 | 0.630707 | 1,31684 | 0.365926 | 1,74037 |
| unit | h | 1/h | h | h | h | h |

EP 0 226 588 B1

## TABLE 5

### PHARMACOKINETIC PARAMETERS AT DAY 5

| Parameter | AUC − 1 | AUC − 3 | $C_{max}$ | $C_{max}$ (m) | $T_{max}$ | $T_{max}$ (m) |
|---|---|---|---|---|---|---|
| **Treatment D** | | | | | | |
| mean | 314.36 | 316.778 | 77.762 | 72.1625 | 1.96585 | 1.86111 |
| sdev | 68.5091 | 70.5779 | 26.6592 | 23.067 | 1.07274 | 0.815981 |
| sem | 19.7769 | 20.3741 | 7.69586 | 6.65886 | 0.309672 | 0.235553 |
| unit | hxng/ml | hxng/ml | ng/ml | ng/ml | h | h |
| **Treatment E** | | | | | | |
| mean | 375.149 | 365.366 | 235.435 | 214.783 | 0.474823 | 0.527778 |
| sdev | 104.109 | 104.008 | 92.2885 | 79.9681 | 0.449741 | 0.475848 |
| sem | 30.0537 | 30.0245 | 26.6414 | 23.0848 | 0.129829 | 0.137365 |
| unit | hxng/ml | hxng/ml | ng/ml | ng/ml | h | h |
| **Treatment F** | | | | | | |
| mean | 254.105 | 253.341 | 45.0807 | 44.6075 | 1.29576 | 1.36111 |
| sdev | 77.0613 | 73.2874 | 13.9579 | 13.7587 | 0.974988 | 0.984305 |
| sem | 22.2457 | 21.1563 | 4.02931 | 3.97181 | 0.281455 | 0.284144 |
| unit | hxng/ml | hxng/ml | ng/ml | ng/ml | h | h |

EP 0 226 588 B1

## TABLE 5 CONTINUED

### PHARMACOKINETIC PARAMETERS AT DAY 5

| Parameter | lag-time | ke | t1/2 | t1/2 alfa | t1/2 beta |
|---|---|---|---|---|---|
| **Treatment D** | | | | | |
| mean | 0.243357 | 1.91282 | 1.65762 | 0.201651 | 4.44231 |
| sdev | 0.198066 | 3.81631 | 1.05178 | 0.26219 | 1.34566 |
| sem | 0.057177 | 1.10167 | 0.303624 | 0.117255 | 0.601799 |
| unit | h | 1/h | h | h | h |
| **Treatment E** | | | | | |
| mean | 0.182272 | 0.778169 | 1.06888 | 0.492011 | 2.20574 |
| sdev | 0.049525 | 0.358517 | 0.516385 | 0.638146 | 0.78898 |
| sem | 0.014297 | 0.103495 | 0.149068 | 0.184217 | 0.227759 |
| unit | h | 1/h | h | h | h |
| **Treatment F** | | | | | |
| mean | 0.052389 | 0.180707 | 12.5622 | 0.892342 | 145.944 |
| sdev | 0.083218 | 0.133566 | 26.9013 | 0.60656 | 305.943 |
| sem | 0.024023 | 0.038557 | 7.76572 | 0.271262 | 136.822 |
| unit | h | 1/h | h | h | h |

## Claims

1. Process for preparation of a solid administration form for oral use based on nifedipine with controlled release of the active substance, characterized by the fact that a mixture of nifedipine crystals having a specific surface area between 0.1 and 0.4 m²/g is prepared, said mixture of crystals is blended with an

EP 0 226 588 B1

equal amount by weight of auxiliary vehicle substances, and the resulting mixture so obtained is applied on spheroids of inert substances, by means of suitable binders, in coating pans.

2. Process according to Claim 1, characterized by the fact that the auxiliary vehicle substances are chosen from the group comprising lactose, corn starch and modified starch.

3. Process according to Claim 1, characterized by the fact that the inert substances for the spheroids are chosen from the group comprising sucrose and starch.

4. Process according to Claim 1, characterized by the fact that the binders are chosen from the group comprising solutions of low molecular weight polyvinylpyrrolidone and solutions of polyoxyethyleneglycol.

5. Process according to Claim 1, characterized by the fact that the coating pan is being rotated at a speed between 10 and 20 rpm and preferably at 16 rpm.

6. Solid administration form for oral use based on nifedipine with controlled release of the active substance, whenever obtained by the process according to one or more of Claims 1-5.

7. Solid administration form for oral use based on nifedipine in the form of crystals having a specific surface area between 0.1 and 0.4 $m^2$/g with controlled release of the active substance for treating arterial hypertension, mixed with auxiliary vehicle substances and applied on spheroids of inert substances with a granulometry between 0.4 and 2 mm

8. Solid administration form for oral use based on nifedipine with controlled release of the active substance according to Claim 7, characterized by the fact that the microgranules are dosed in hard gelatine capsules in an amount of 5 to 50 mg of nifedipine.

9. A pharmaceutical composition comprising inert granules having a coating of particulate nifedipine with a surface area between 0.1 and 0.4 $m^2$/g, and a binding agent.

10. A composition as claimed in Claim 9 wherein the coating contains a particulate excipient.

11. A composition as claimed in claim 9 or 10 wherein the excipient is lactose.

12. A composition as claimed in anyone of claims 9 to 11, wherein the binding agent is low molecular weight polyoxyethylene glycol.

13. A composition as claimed in anyone of claims 9 to 11 wherein the binding agent is low molecular weight polyvinylpyrrolidone.

14. A composition as claimed in anyone of claims 9 to 13 wherein the granule is sucrose and corn starch.

15. A capsule containing a composition as claimed in anyone of claims 9 to 14

16. A process for preparing a composition as claimed in anyone of claims 9 to 15 which comprises the steps of coating the inert granules with the particulate nifedipine and the binding agent and optionally filling the composition into a capsule shell.

**Revendications**

1. Procédé de préparation d'une forme d'administration solide, destinée à l'utilisation orale, à base de nifédipine, permettant une libération contrôlée de la substance active, caractérisé en ce que l'on prépare un mélange de cristaux de nifédipine dont la surface spécifique est comprise entre 0,1 et 0,4 $m^2$/g, ledit mélange de cristaux étant mélangé à une quantité égale en poids de substances véhiculaires auxiliaires et le mélange ainsi obtenu étant appliqué sur des sphéroïdes de substances inertes, au moyen de liants adéquats, dans des cuves d'enrobage.

2. Procédé selon la revendication 1, caractérisé en ce que les substances véhiculaires auxiliaires sont choisies dans le groupe constitué par le lactose, l'amidon de maïs et l'amidon modifié.

3. Procédé selon la revendication 1, caractérisé en ce que les substances inertes formant la base des sphéroïdes sont choisies dans le groupe constitué par le sucrose et l'amidon.

4. Procédé selon a revendication 1, caractérisé en ce que les liants sont choisis dans le groupe constitué par des solutions de polyvinylpyrrolidone de faible poids moléculaire et des solutions de polyoxyéthylèneglycol.

5. Procédé selon la revendication 1, caractérisé en ce que l'on fait tourner la cuve d'enrobage à une vitesse comprise entre 10 et 20 tr/mn, de préférence à 16 tr/mn.

6. Forme d'administration solide destinée à l'utilisation par voie orale à base de nifédipine permettant une libération contrôlée de la substance active obtenue par le procédé selon l'une quelconque ou plusieurs des revendications 1 à 5.

7. Forme d'administration solide destinée à l'utilisation par voie orale à base de nifédipine sous forme de cristaux dont la surface spécifique est comprise entre 0,1 et 0,4 $m^2/g$, permettant une libération contrôlée de la substance active en vue du traitement de l'hypertension artérielle, mélangée avec des substances véhiculaires auxiliaires et appliquée sur des sphéroïdes de substances inertes d'une granulométrie comprise entre 0,4 et 2 mm.

8. Forme d'administration solide destinée à l'utilisation par voie orale à base de nifédipine permettant une libération contrôlée de la substance active selon la revendication 7, caractérisée en ce que les microgranules sont introduits dans des capsules de gélatine dure par quantité de 5 à 50 mg de nifédipine.

9. Composition pharmaceutique comprenant des granulés inertes enrobés de nifédipine particulaire dont la surface spécifique est comprise entre 0,1 et 0,4 $m^2/g$, et un agent liant.

10. Une composition selon la revendication 9, dans laquelle l'agent d'enrobage comprend un excipient sous forme de particules.

11. Une composition selon la revendication 9 ou 10, dans laquelle l'excipient est le lactose.

12. Une composition selon l'une quelconque des revendications 9 à 11, dans laquelle l'agent liant est un polyoxyéthylèneglycol de faible poids moléculaire.

13. Une composition selon l'une quelconque des revendications 9 à 11, dans laquelle l'agent liant est une polyvinylpyrrolidone de faible poids moléculaire.

14. Une composition selon l'une quelconque des revendications 9 à 13, dans laquelle le granulé est à base de sucrose et d'amidon de maïs.

15. Une capsule contenant une composition selon l'une quelconque des revendications 9 à 14.

16. Un procédé de préparation d'une composition selon l'une quelconque des revendications 9 à 15, qui consiste à enrober les granulés inertes de nifédipine particulaire et d'agent liant et, éventuellement, à introduire cette composition dans une capsule.

**Ansprüche**

1. Verfahren zur Herstellung einer festen Verabreichungsform zur oralen Anwendung auf der Basis von Nifedipin mit einer gesteuerten Freisetzung der Wirksubstanz, dadurch gekennzeichnet, daß eine Mischung von Nifedipinkristallen mit einer spezifischen Oberfläche zwischen 0,1 und 0,4 $m^2/g$ hergestellt wird, diese Kristallmischung mit einer gleichen Gewichtsmenge von Träger-Hilfsstoffen vermischt wird und die auf diese weise erhaltene fertige Mischung mit Hilfe geeigneter Bindemittel in Dragierkesseln auf Sphäroide aus Inertsubstanzen aufgebracht wird.

2.  Verfahren nach Anspruch 1, dadurch gekennzeichnet, daß die Trager-Hilfsstoffe aus der Gruppe ausgewahlt sind, die Lactose, Maisstarke und modifizierte Stärke umfaßt.

3.  Verfahren nach Anspruch 1, dadurch gekennzeichnet, daß die Inertsubstanzen für die Sphäroide aus der Gruppe ausgewahlt sind, die Saccharose und Stärke umfaßt.

4.  Verfahren nach Anspruch 1, dadurch gekennzeichnet, daß die Bindemittel aus der Gruppe ausgewählt sind, die Lösungen von niedermolekulargewichtigem Polyvinylpyrrolidon und Lösungen von Polyoxyethylenglycol umfaßt.

5.  Verfahren nach Anspruch 1, dadurch gekennzeichnet, daß der Dragierkessel mit einer Rotationsgeschwindigkeit zwischen 10 und 20 U/min, vorzugsweise mit 16 U/min, betrieben wird.

6.  Fegte Verabreichungsform für die orale Anwendung auf der Basis von Nifedipin mit gesteuerter Freisetzung des Wirkstoffs, wenn sie nach dem Verfahren nach einen oder mehreren der Ansprüche 1 bis 5 erhalten wurde.

7.  Feste Verabreichungsform zur oralen Anwendung auf der Basis von Nifedipin in Form von Kristallen mit einer spezifischen Oberfläche zwischen 0,1 und 0,4 $m^2/g$ mit einer gesteuerten Freisetzung des Wirktoffs zur Behandlung von arterieller Hypertonie, gemischt mit Träger-Hilfsstoffen und aufgebracht auf spharoide aus Inertsubstanzen mit einer Granulometrie zwischen O,4 und 2 mm.

8.  Feste Verabreichungsform zur oralen Anwendung auf der Basis von Nifedipin mit gesteuerter Freisetzung des Wirkstoffs nach Anspruch 7, dadurch gekennzeichnet, daß die Mikrogranulatteilchen in einer Menge von 5 bis 50 mg Nifedipin in Hartgelatinekapseln eindosiert sind.

9.  Pharmazeutische Zusammensetzung mit inerten Granulatteilchen mit einem Überzug aus teilchenförmigem Nifedipin mit einer Oberfläche zwischen O,1 und 0,4 $m^2/g$ und einem Bindemittel.

10.  Zusammensetzung nach Anspruch 9, bei der der Überzug einen teilchenförmigen Hilfsstoff enthält.

11.  Zusammensetzung nach Anspruch 9 oder 10 bei der der Hilfsstoff Lactose ist.

12.  Zusammensetzung nach irgendeinem der Ansprüche 9 bis 11, bei der das Bindemittel niedermolekulargewichtiges Polyoxyethylenglycol ist.

13.  Zusammensetzung nach irgendeinem der Ansprüche 9 bis 11, bei der das Bindemittel niedermolekulargewichtiges Polyvinylpyrrolidon ist.

14.  Zusammensetzung nach irgendeinem der Ansprüche 9 bis 13, bei der das Granulat Saccharose oder Maisstärke ist.

15.  Ein Kapsel, die eine Zusammensetzung nach irgendeinem der Ansprüche 9 bis 14 enthält.

16.  Verfahren zur Herstellung einer Zusammensetzung nach irgendeinem der Ansprüche 9 bis 15, dadurch gekennzeichnet, daß es die Stufen des Überziehens des inerten Granulats mit dem teilchenformigen Nifedipin und dem Dindemittel sowie gegebenenfalls das Einfüllen der Zusammensetzung in eine Kapselhülle umfaßt.

PLASMA LEVELS OF NIFEDIPINE

mean over subjects

FIG. 1

PLASMA LEVELS OF NIFEDIPINE
mean over subjects

FIG. 2

EP 0 226 588 B1

*Fig.3*

PERCENT

NIFEDIPINE EXAMPLE A 300 g METHACRYLATES

△ NIFEDIPINE EXAMPLE A 200 g METHACRYLATES

_Fig.4_

NIFEDIPINE OF EXAMPLE B

PLASMA LEVELS OF NIFEDIPINE

mean over subjects FIG. 5

Concentration (ng/ml)

Treatment A
measured

Treatment B
measured

Treatment C
measured

Time (h)

EP 0 226 588 B1

PLASMA LEVELS OF NIFEDIPINE

mean over subjects (elimin. phase) FIG. 6

Concentration (ng/ml)

Treatment D measured

Treatment E measured

Treatment F measured

Time (h)